# EUROPEAN PATENT SPECIFICATION

(11) **EP 2 405 926 B1**
(45) Date of publication and mention of the grant of the patent: **20.07.2016**
(21) Application number: 10710670.0
(22) Date of filing: 10.03.2010
(51) Int. Cl.: A61K 35/74, A61K 36/815, A61K 31/07, A61K 31/715, A61K 8/97, A61K 8/99, A61Q 5/00, A61P 17/00

(54) **COSMETIC USE OF AN EXTRACT OF WOLFBERRY FOR TREATING SCALP DISORDERS**
KOSMETISCHE ANWENDUNG EINES BOCKSDORN-EXTRAKTS ZUR BEHANDLUNG VON ERKRANKUNGEN DER KOPFHAUT
UTILISATION COSMÉTIQUE D'UN EXTRAIT DE SYMPHORINE OCCIDENTALE POUR TRAITEMENT DE TROUBLES DU CUIR CHEVELU

(30) Priority: 11.03.2009 FR 0901148; 17.03.2009 FR 0951694; 24.04.2009 US 202989 P
(43) Date of publication of application: 18.01.2012
(73) Proprietor: L'Oréal, 75008 Paris (FR); Nestec S.A., 1800 Vevey (CH)
(72) Inventor: CASTIEL, Isabelle, F-06200 Nice (FR); GUENICHE, Audrey, F-92500 Rueil Malmaison (FR)
(74) Representative: Nony
(86) International application number: PCT/IB2010/051034
(87) International publication number: WO 2010/103472

(56) References cited:
- EP-A- 1 616 551
- EP-A1- 2 174 648
- WO-A-87/03803
- WO-A-2008/067315
- WO-A2-2010/056675
- FR-A1- 2 920 304
- DATABASE WPI Week 200279 Thomson Scientific, London, GB; AN 2002-729338 XP002545087 & KR 2002 029 455 A (HWANG I M) 19 April 2002 (2002-04-19)
- DATABASE WPI Week 20098 Thomson Scientific, London, GB; AN 2009-B30760 XP002545088 & KR 10 822 835 A (SAIMDANG COSMETICS CO LTD) 16 April 2008 (2008-04-16)
- BENZIE IRIS F F ET AL: "ENHANCED BIOAVAILABILITY OF ZEAXANTHIN IN A MILK-BASED FORMULATION OF WOLFBERRY (GOU QI ZI; FRUCTUS BARBARUM L.)" BRITISH JOURNAL OF NUTRITION, CAMBRIDGE UNIVERSITY PRESS, CAMBRIDGE, GB, vol. 96, no. 1, 1 January 2006 (2006-01-01), pages 154-160, XP009072766 ISSN: 0007-1145

## Description

Herein is described a novel active agent for the prevention and/or treatment of scalp disorders, especially aesthetic scalp disorders, including dandruff disorders of the scalp.

The present invention aims to propose a novel active agent for the prevention and/or treatment of inflammation-related scalp disorders, especially aesthetic scalp disorders. In particular, the present invention relates to inflammation-related dandruff disorders of the scalp.

The present invention relates to the field of topical products, of dietary supplements or of functional foods intended for scalp care.

The scalp is an epidermis that undergoes continual renewal, like the rest of the skin tissue, and that is rich in sebaceous glands.

Normally, the scalp undergoes renewal by removing the surface cells of the skin, without this renewal being felt or seen. However, excessive renewal of the cells of the *stratum corneum* of the scalp, for various reasons, may lead to the formation of large, thick clumps of cells that are visible to the naked eye, known as "dandruff".

Dandruff conditions are recurring, frequent, chronic conditions which are socially handicapping owing to their clearly unattractive nature.

Various factors may promote the onset of dandruff. For example, mention may be made of stress, the winter period, a lack of hydration, or colonization of the skin or of the hair follicles by the yeast *Malassezia sp..* These factors especially have the common feature of causing and/or promoting an inflammatory skin condition. Such an inflammation reinforces the appearance or even increases the presence of dandruff.

These dandruff conditions and/or inflammatory conditions of the scalp are reflected by an impairment in the barrier function of the epidermis. What is more, these conditions may give rise to sensations of itching or pruritus, leading to scratching behavior that amplifies the phenomenon of appearance of the dandruff.

The dandruff conditions of the scalp may be of greasy type or of dry type.

The dry dandruff conditions of the scalp are more frequently manifested and are amplified during skin hydration disorders, and especially during substantial dryness of the scalp epidermis.

Moreover, the scalp is rich in sebaceous glands. It has been observed that hypersecretion of sebum, or seborrhea, may lead to disruptions, sensations of discomfort, aesthetic disorders, or even a pathological skin condition. Thus, excessive secretion of sebum promotes the appearance of a greasy dandruff condition of the scalp or greasy dandruff. Moreover, said dandruff has a tendency to be more readily pruritic.

Dandruff conditions generally respond to various local or systemic antifungal treatments. However, the efficacy of these treatments is only suspensory and demands rigorous adherence on the part of the user (sufficient frequency of use and sufficient application time).

Consequently, many failures arise in the use of these treatments and can usually be attributed to the following factors: incorrect following of the protocol; frequency of use not adhered to; noncosmetic appearance of the product; irritation by the washing base; poor adherence to the application time; lassitude.

There is thus still a need for novel active agents capable of exerting a beneficial cosmetic or therapeutic action on scalp conditions.

There is also a need for novel active agents capable of exerting a preventive and/or care action against inflammation-related dandruff conditions which is long-lasting.

There is also a need for novel active agents for preventing and/or treating inflammatory conditions of the scalp.

The object of the present invention is to meet these needs.

Thus, according to a first subject, the invention relates to the cosmetic use of an effective amount of an extract of wolfberry obtained by extraction of wolfberry fruit of at least one plant of the species *Lycium barbarum* in a liquid medium containing milk or milk proteins, in particular of lacto-wolfberry, as an agent for preventing and/or treating aesthetic scalp disorders, and wherein the aesthetic scalp disorder is an inflammation-related dandruff condition of the scalp.

Unexpectedly, the inventors have observed that an extract of wolfberry obtained by extraction of wolfberry fruit of at least one plant of the species *Lycium barbarum* in a liquid medium containing milk or milk proteins, in particular of lacto-wolfberry, makes it possible to reduce dandruff conditions by acting on the inflammation and on the barrier function of the scalp.

The use of such an extract promotes a reduction in or even inhibits the expression and the release of inflammatory cellular factors, such as cytokines or enzymes, thus preventing the impairment of the various functional and structural elements of the skin.

Thus, reducing inflammatory conditions of the skin, and in particular of the scalp, makes it possible to reinforce the barrier functions of the skin, and to maintain their equilibrium and their integrity.

The scalp is then less irritated and pruriginous, less fragile and more hydrated, and the dandruff conditions are reduced.

Thus, the use of an extract of wolfberry according to the invention, in particular of lacto-wolfberry, makes it possible to restore a normal scalp, in perfect homeostasis.

According to another advantage, a use according to the invention can reduce and/or treat pruritus of the scalp subsequent to the presence of irritant or inflammatory metabolites resulting from aggressive external or intrinsic factors such as stress, the winter period, a hydration deficiency or colonization of the skin or of the hair follicles by the yeast *Malassezia sp.*

According to another advantage, a use according to the invention can promote hydration and maintenance of the integrity of the scalp.

Herein is described the cosmetic use of an effective amount of an extract of wolfberry as an agent for improving the comfort of the scalp.

Herein is also described the cosmetic use of an effective amount of an extract of wolfberry as an agent for scalp hygiene and/or care.

Herein is also described the cosmetic use of an effective amount of an extract of wolfberry as an agent for preserving and/or reinforcing the integrity of the barrier functions of the scalp.

According to another of its aspects, the present invention relates to the cosmetic use of an effective amount of an extract of wolfberry as an agent for preventing and/or treating pruritus and/or seborrheic dermatitis of the scalp.

According to another of its aspects, the present invention relates to an effective amount of at least one extract of wolfberry obtained by extraction of wolfberry fruit of at least one plant of the species *Lycium barbarum* in a liquid medium containing milk or milk proteins, in particular of lacto-wolfberry, for use in preventing and/or treating inflammation of the scalp.

In particular, such a composition is found to be effective for treating dry conditions or xerosis of the scalp, pruritus of the scalp or seborrheic dermatitis of the scalp.

For the purpose of the present invention, the term "prevent" means completely eliminate or partially reduce the risk of manifestation of the phenomenon concerned, i.e., in the second case, the risk remains, but to a lesser degree than before the use of the invention.

A method of the invention is in particular advantageously used in individuals having an inflammation-related dandruff condition of the scalp.

A use in accordance with the invention may, in addition, comprise the use of at least an effective amount of at least one extract of wolfberry obtained by extraction of wolfberry fruit of at least one plant of the species *Lycium barbarum* in a liquid medium containing milk or milk proteins, in particular of lacto-wolfberry, in combination with an effective amount of at least one probiotic microorganism, in particular of the *Lactobacillus sp.* genus, and/or a fraction thereof, and/or a metabolite thereof.

According to another of its aspects, the present invention relates to a cosmetic and/or dermatological composition which is of use for preventing and/or treating an inflammation of the scalp, in particular inflammation-related dandruff conditions of the scalp, comprising, in a physiologically acceptable medium, at least an effective amount of at least one extract of wolfberry obtained by extraction of wolfberry fruit of at least one plant of the species *Lyceum barbarum* in a liquid medium containing milk or milk proteins, in particular of lacto-wolfberry, optionally in combination with an effective amount of at least one active agent chosen from an antiseborrheic active agent, a moisturizing active agent, an antidandruff active agent, and mixtures thereof, in particular as described hereinafter.

According to one variant embodiment, a use according to the invention may be carried out orally.

According to one variant embodiment, a use according to the invention may be carried out topically.

As specified hereinafter, the compositions used according to the invention are formulated so as to be compatible with the method of administration selected.

An extract of wolfberry in accordance with the invention may be in the form of a cosmetic or dermatological or pharmaceutical composition.

For the purpose of the present invention, the term "effective amount" is intended to mean an amount sufficient to obtain the expected effect.

### Dandruff conditions

As indicated previously, a scalp exhibiting excessive dryness or excessive sebum secretion may show a dandruff condition which, depending on the case, will be characterized by the presence of dry or greasy dandruff flakes, or even pruritus and/or inflammation of the epidermis.

Dry dandruff conditions reflect a xerosis of the scalp, combined, as appropriate, with excessively rapid renewal of its *stratum corneum.* Dry dandruff flakes are generally small and white or gray, and are spread over the scalp and on clothing, giving rise to an unaesthetic visual effect.

The itching associated with dryness of the scalp may lead to erythema, pruritus or even inflammation.

Greasy dandruff conditions are one of the forms of seborrheic dermatitis.

Individuals suffering therefrom have an erythematous scalp covered with large, greasy yellow squamae that accumulate to form packets. They have a pruritic scalp, and often have burning sensations on the affected areas.

During dandruff conditions of the scalp, the skin barrier is unbalanced, its integrity and its hydration are impaired and its ecoflora disrupted. The skin of the scalp is irritated and pruritic, fragile, less hydrated, and sensitive to infection.

The use of an extract of wolfberry in accordance with the invention, in particular of lacto-wolfberry, leads to a reduction in inflammation, to re-establishment of the hydration and of the ecoflora and to a decrease in the pruritus of the scalp. The reduction in inflammation and the decrease in pruritus result in a reduction in the phases of scratching of the scalp and in the impairment of the barrier function resulting therefrom.

The skin is then less irritated and less pruritic and the presence of dandruff is reduced, or even eliminated.

The uses, methods and compositions according to the invention thus prove to be most particularly effective on inflammation of the scalp:
- for preventing and/or treating inflammation-related disorders of the scalp, especially inflammation-related aesthetic disorders of the scalp associated with excessive dryness, or even xerosis,
- for preventing and/or treating inflammation-related disorders of the scalp, especially inflammation-related aesthetic disorders of the scalp associated with excessive sebum excretion and/or secretion,
- for preventing and/or treating inflammation-related disorders, especially inflammation-related aesthetic disorders of the scalp, in particular a dandruff condition, whether it is dry or greasy, of the scalp,
- for improving the comfort of the scalp,
- for improving the hygiene and/or the care of the scalp,
- for giving the scalp a feeling of well-being,
- for preserving and/or reinforcing the integrity of the barrier functions of the scalp,
- for maintaining and/or restoring the biomechanical properties of the scalp,
- for re-establishing a balanced ecoflora of the scalp,
- for preventing and/or treating pruritus and/or seborrheic dermatitis of the scalp,
- for preventing and/or treating inflammatory conditions of the scalp.

In particular, an extract of wolfberry according to the invention may be more particularly devoted to preventing and/or treating an inflammation-related dandruff condition of the scalp.

### Extract of wolfberry

For the purpose of the invention, the term "wolfberry" is intended to denote the species of the *Lycium* genus which are chosen from *Lycium barbarum* and *Lycium chinense,* and preferably the species *Lycium barbarum.* Extacts from L. barbarum obtained by water, ethanol and/or ethyl acetate extraction for use in dermatological disorders are known from WO 2008/067315 and WO 87/03803.

The extract of wolfberry according to the invention is obtained by extraction of wolfberry fruit of at least one plant of the species *Lycium barbarum* in a liquid medium containing milk or milk proteins.

An extract of wolfberry under consideration according to the invention contains at least the essential lipophilic and/or hydrophilic bioactive components of the wolfberry. The term "bioactive" denotes molecules or components having a biological activity, especially on the scalp and in particular with regard to dandruff conditions, when they are administered orally or topically.

According to one embodiment, the essential lipophilic and/or hydrophilic bioactive components are preferably chosen from lipids, alkaloids, proteins, sugars, glycoproteins, polyphenols such as flavonoids, vitamins and minerals, or mixtures thereof.

Carotenoids may be chosen from carotenes and xanthophylls, such as lycopene, carotene, phytofluene, phytoene, canthaxanthin, beta-cryptoxanthin, capsanthin, lutein, and zeaxanthin, fatty acid esters thereof, or mixtures thereof.

The glycoproteins are preferably chosen from arabinogalactans, in particular from *Lycium barbarum* polysaccharides and the macromolecules which can be detected by the beta-glucosyl Yariv reagent.

The flavonoids are preferably chosen from flavones such as apigenin, luteolin or diosmetin, flavonols such as quercetin or myricetin, kaempferol, flavanones, anthocyanidins such as pelargonidin or malvidin, or isoflavones such as genistein or daidzenin, or mixtures thereof.

An extract of wolfberry under consideration according to the invention contains at least zeaxanthin or a derivative thereof and/or *Lycium barbarum* polysaccharide (LBP).

*Lycium barbarum* polysaccharide (LBP) is a group of water-soluble polysaccharides of arabinogalactan protein (AGP) type.

By way of zeaxanthin derivative, mention may in particular be made of zeaxanthin esters and diesters, and more particularly zeaxanthin dipalmitate.

Thus, the wolfberry fruit naturally comprises a zeaxanthin content (including zeaxanthin dipalmitate and other zeaxanthin derivatives) ranging from 20 to 300 mg, and preferably from 50 to 250 mg by weight, in particular from 100 to 200 mg by weight per 100 g of fruit.

The wolfberry fruit also contains vitamin C or an analog thereof.

By way of vitamin C analog, mention may in particular be made of ascorbyl glucoside, and, for example, 2-O-(β-D-glucopyranosyl)ascorbic acid.

According to one embodiment, the extract of wolfberry used according to the invention contains at least zeaxanthin dipalmitate and *Lycium barbarum* polysaccharide.

According to one embodiment, the extract of wolfberry used according to the invention contains at least zeaxanthin dipalmitate, *Lycium barbarum* polysaccharide (LBP) and 2-O-(β-D-glucopyranosyl)ascorbic acid.

In particular, this extract may advantageously contain up to 0.30% by weight, for example from 0.01 to 0.30% by weight, in particular from 0.03% to 0.12% by weight, or even from 0.06% to 0.10% by weight of zeaxanthin or derivatives, including zeaxanthin dipalmitate and other zeaxanthin derivatives, relative to its total weight.

The extract of wolfberry is an extract obtained by extraction of wolfberry fruit of at least one plant of the species *Lycium barbarum*, said material having been obtained *by in vitro or in vivo* culture.

It is possible to use, as extraction solvent, a milk medium as described hereinafter.

According to a preferred embodiment of the invention, an extract that can be prepared according to one of the methods described in document WO 2005/092121 is used.

An extract of wolfberry obtained by extraction of wolfberry fruit of at least one plant of the *Lycium barbarum* species in a liquid medium containing milk or milk proteins, is used.

According to this embodiment, a milk extract of wolfberry or lacto-wolfberry can, in particular, be obtained by means of a method of preparation comprising the steps consisting in mixing and grinding the whole wolfberry fruit in a liquid medium containing milk or milk proteins, optionally separating the insoluble fiber so as to obtain an aqueous suspension, and optionally drying out the suspension thus isolated so as to obtain a powder.

Where appropriate, a pasteurization step may be carried out on the aqueous suspension obtained after separation of the insoluble fiber, and before the step consisting in drying out the suspension so as to obtain a powder.

The steps of mixing, grinding and separating the insoluble fiber can be carried out at ambient temperature and at atmospheric pressure.

Such a method makes it possible to advantageously extract the essential hydrophilic and lipophilic bioactive components of wolfberry, in particular as defined above.

Such a milk extract can also be described as lacto-wolfberry.

The milk or the milk proteins suitable for the preparation of lacto-wolfberry, or milk extract of wolfberry, may be of animal or plant origin, or be a mixture thereof. In particular, the milk may be cow's milk, llama's milk, buffalo's milk, goat's milk, ewe's milk, or a plant milk such as soya milk, or a mixture thereof. The term "milk protein" denotes a protein fraction derived from one or more of the milks mentioned above.

Advantageously, cow's milk or soya milk may be used.

Plant oils may optionally be added to the milk

The wolfberry fruit can be mixed and ground in the milk or a liquid containing milk proteins, according to a weight ratio ranging from 1:1 to 1:1000, and preferably from 1:5 to 1:50.

The mixing and the grinding can be carried out at a temperature ranging from 1 to 95°C, preferably from 20 to 80°C, and more particularly from 40 to 80°C.

Next, the insoluble fiber can be at least partially removed so as to obtain an aqueous suspension.

Preferably, the milk extract of wolfberry constitutes a miscible composition.

According to one embodiment, the extract of wolfberry can be in the form of a water-dispersible powder. This formulation in fact makes it possible to increase the bioavailability of the zeaxanthin of the extract of wolfberry.

The extract of wolfberry according to the invention, and more particularly the lacto-wolfberry, is present in an effective amount for conferring, on the compositions in which it is formulated, the required properties according to the invention.

Thus, the cosmetic or dermatological compositions of the invention can comprise an amount, in terms of solids, of extract of wolfberry ranging from 0.5% to 20% by weight, relative to the total weight of the composition comprising it.

For obvious reasons, this amount of extract of wolfberry can vary to a large extent and can depend in particular on the desired activity and/or on the method of administration, i.e. topical or oral administration, selected for the corresponding composition.

For example, the extract of wolfberry according to the invention, and more particularly the lacto-wolfberry, can be used in an amount, in terms of solids, ranging from 0.5% to 20% by weight, and in particular in a proportion of at least 5% by weight, preferably of at least 10% by weight, relative to the total weight of the composition according to the invention comprising it, and more particularly in those intended for oral application.

According to a variant embodiment of the invention, an extract of wolfberry of the invention can be advantageously combined with at least an effective amount of at least one microorganism, in particular probiotic microorganism, of a fraction thereof or of a metabolite thereof.

### Microorganisms

According to one embodiment, an extract of wolfberry of the invention is advantageously used in combination with at least one probiotic microorganism, in particular a lactic acid probiotic microorganism.

For the purpose of the present invention, the term "probiotic microorganism" is intended to mean a live microorganism which, when it is consumed in adequate amount, has a positive effect on the health of its host ("Joint FAO/WHO Expert Consultation on Evaluation of Health and Nutritional Properties of Probiotic in Food Including Powder Milk with Live Lactic Acid Bacteria, October 6, 2001"), and which can in particular improve the intestinal microbial balance.

According to one variant of the invention, this microorganism is used in an isolated form, i.e. not mixed with one or more compound(s) that may be associated with it in its medium of origin.

For the purpose of the invention, the term "metabolite" denotes any substance derived from the metabolism of the microorganisms under consideration according to the invention and also having efficacy in the treatment of greasy or dry scalps.

For the purpose of the invention, the term "fraction" denotes more particularly a fragment of said microorganism, which has efficacy in the treatment of scalps by analogy with said whole microorganism.

A probiotic microorganism suitable for the invention may be chosen in particular from ascomycetes such as *Saccharomyces*, *Yarrowia, Kluyveromyces*, *Torulaspora, Schizosaccharomyces pombe, Debaromyces*, *Candida, Pichia, Aspergillus* and *Penicillium*, and bacteria of the genus *Bifidobacterium, Bacteroides, Fusobacterium, Melissococcus, Propionibacterium, Enterococcus, Lactococcus, Staphylococcus, Peptostrepococcus, Bacillus, Pediococcus*, *Micrococcus, Leuconostoc, Weissella, Aerococcus, Oenococcus* and *Lactobacillus*, and mixtures thereof.

A lactic acid probiotic microorganism suitable for the invention may particularly be chosen from the microorganisms *Lactobacillus johnsonii, Lactobacillus reuteri, Lactobacillus rhamnosus*, *Lactobacillus paracasei, Lactobacillus casei, Bifidobacterium bifidum, Bifidobacterium breve, Bifidobacterium longum, Bifidobacterium animals, Bifidobacterium lactis, Bifidobacterium infantis, Bifidobacterium adolescentis* and *Bifidobacterium pseudocatenulatum,* a fraction thereof or a metabolite thereof, and mixtures thereof.

The species most particularly suitable are *Lactobacillus johnsonii, Lactobacillus paracasei, Bifidobacterium adolescentis* and *Bifidobacterium longum,* a fraction thereof or a metabolite thereof, respectively deposited according to the Treaty of Budapest with the Institut Pasteur (28 rue du Docteur Roux, F-75024 Paris cedex 15) on 06/30/92, 01/12/99, 04/15/99 and 04/15/99 under the following designations: CNCM I-1225, CNCM I-2116, CNCM I-2168 and CNCM I-2170, and *Bifidobacteriurn lactis (Bb* 12) (ATCC27536) or *Bifidobacterium longum (BB536),* a fraction thereof or a metabolite thereof. The *Bifidobacterium lactis (ATCC27536)* strain can be obtained from Hansen (Chr. Hansen A/S, 10-12 Boege Alle, P.O. Box 407, DK-2970 Hoersholm, Denmark).

According to one embodiment, a probiotic microorganism suitable for the invention may in particular be a microorganism of the *Lactobacillus sp.* genus, a fraction thereof or a metabolite thereof.

Preferably, a microorganism of the *Lactobacillus sp.* genus that is suitable for the invention may be chosen from the species *Lactobacillus johnsonii, Lactobacillus reuteri, Lactobacillus paracasei* and *Lactobacillus casei,* a fraction thereof or a metabolite thereof, and mixtures thereof.

According to one preferred embodiment, a probiotic microorganism suitable for the invention may be *Lactobacillus paracasei,* a fraction thereof or a metabolite thereof

According to one even more preferred embodiment, a probiotic microorganism suitable for the invention may be the *Lactobacillus paracasei* ST11 strain deposited according to the Treaty of Budapest with the Institut Pasteur (28 rue du Docteur Roux, F-75024 Paris cedex 15) on January 12, 1999 under the designation CNCM I-2116, and/or a fraction thereof and/or a metabolite thereof.

According to one variant embodiment, in addition to a first probiotic microorganism as defined above, the invention may comprise the use of at least an effective amount of at least a second microorganism, in particular of probiotic type, and/or a fraction thereof and/or a metabolite thereof, said second microorganism being different than said first microorganism.

This second microorganism may be chosen in particular from ascomycetes such as *Saccharomyces, Yarrowia, Kluyveromyces, Torulaspora, Schizosaccharomyces pombe, Debaromyces, Candida, Pichia, Aspergillus* and *Penicillium,* and bacteria of the genus *Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus, Lactobacillus* and *Bifidobacterium,* and mixtures thereof.

As ascomycetes that are most particularly suitable for the present invention, mention may in particular be made of *Yarrowia lipolitica* and *Kluyveromyces lactis,* just as it may be made of *Saccharomyces cereviseae, Torulaspora, Schizosaccharomyces pombe, Candida* and *Pichia.*

Specific examples of probiotic second microorganisms suitable for the invention are *Bifidobacterium adolescentis, Bifidobacterium animalis, Bifidobacterium bifidum, Bifidobacterium breve, Bifidobacterium lactis, Bifidobacterium longum, Bifidobacterium infantis, Bifidobacterium pseudocatenulatum, Lactobacillus acidophilus* (NCFB 1748); *Lactobacillus amylovorus, Lactobacillus casei (Shirota), Lactobacillus rhamnosus* (strain GG), *Lactobacillus brevis, Lactobacillus crispatus, Lactobacillus delbrueckii (subsp bulgaricus, lactis), Lactobacillus fermentum, Lactobacillus helveticus, Lactobacillus gallinarum, Lactobacillus gasseri, Lactobacillus johnsonii* (CNCM I-1225), *Lactobacillus plantarum, Lactobacillus reuteri, Lactobacillus salivarius, Lactobacillus alimentarius, Lactobacillus curvatus, Lactobacillus casei subsp. casei, Lactobacillus sake, Lactococcus lactis, Enterococcus (faecalis, faecium), Lactococcus lactis (subsp lactis* or *crernoris), Leuconostoc mesenteroides* subsp *dextranicum, Pediococcus acidilactici, Sporolactobacillus inulinus, Streptococcus salvarius subsp. thermophilus, Streptococcus thermophilus, Staphylococcus carnosus, Staphylococcus xylosus, Saccharomyces (cerevisiae* or else *boulardii), Bacillus (cereus var toyo* or *subtilis), Bacillus coagulans, Bacillus licheniformis, Escherichia coli strain nissle* and *Propionibacterium freudenreichii,* and mixtures thereof.

More particularly, they may be probiotic microorganisms derived from the group of lactic acid bacteria, such as, in particular, *Lactobacillus* and/or *Bifidobacterium.*

According to one embodiment, as second microorganism, the following bacterial yeast genera are preferentially used:
- lactic acid bacteria: which produce lactic acid by fermentation of sugar. Depending on their morphology, they are divided up into two groups:
   *Lactobacillus species: Lactobacillus acidophilus, amylovorus, casei, rhamnosus, brevis, crispatus, delbrueckii (subsp bulgaricus, lactis),fermentum, helveticus, gallinarum, gasseri, johnsonii, plantarum, reuteri, salivarius, alimentarius, curvatus, casei subsp. casei, sake,* and
   *Cocci : Enterococcus (faecalis, faecium), Lactococcus lactis (subs lactis* or *cremoris), Leuconostoc mesenteroides* subsp *dextranicum, Pediococcus acidilactici, Sporolactobacillus inulinus, Streptococcus salvarius subsp. thermophilus, Streptococcus thermophilus, Staphylococcus carnosus, Staphylococcus xylosus,*
- bifidobacteria or *Bifidobacterium species: Bifidobacterium adolescentis, animalis, bifidum, breve, lactis, longum, infantis, pseudocatenulatum,*
- yeasts: *Saccharomyces (cerevisiae* or else *boulardii),*
- the other sporulated bacteria: *Bacillacs (cereus var toyo* or *subtilis), Bacillus coagulans, Bacillus licheniformis, Escherichia coli strain nissle, Propionibacterium freudenreichii,*
- and mixtures thereof.

More particularly, the second microorganism may be one of the probiotic microorganisms proposed above by way of specific example of probiotic microorganisms for the first organism.

A microorganism of the invention may be formulated in a composition in a proportion of at least 0.0001% (expressed by dry weight), in particular in a proportion of 0.0001 to 20%, and more particularly in a proportion of from 0.001% to 15% by weight, in particular from 0.01% to 10% by weight, and especially from 0.1% to 2% by weight, relative to the total weight of the composition.

In general, a composition according to the invention, and in particular that intended to be administered orally, may comprise, for live microorganisms, from 10³ to 10¹⁵ cfu/g, in particular from 10⁵ to 10¹⁵ cfu/g, and more particularly from 10⁷ to 10¹² cfu/g of microorganisms per gram of support, or equivalent doses calculated for inactive or dead microorganisms or for microorganism fractions or for metabolites produced.

In the particular case of the compositions that must be administered orally, the concentration of each microorganism and/or corresponding fraction and/or corresponding metabolite can be adjusted so as to correspond to doses (expressed as microorganism equivalent) ranging from 5 × 10⁵ to 10¹³ cfu/d, and in particular from 10⁸ to 10¹¹ cfu/d.

A composition for topical application according to the invention may generally comprise from 10³ to 10¹² cfu/g, in particular from 10⁵ to 10¹⁰ cfu/g, and more particularly from 10⁷ to 10⁹ cfu/g of microorganisms.

When a composition comprises metabolites, the metabolite contents in the compositions correspond substantially to the contents capable of being produced by 10³ to 10¹⁵ cfu, in particular 10⁵ to 10¹⁵ cfu, and more particularly 10⁷ to 10¹² cfu of live microorganisms per gram of support.

The expression of the amount of metabolites or of fractions of a microorganism in "cfu", or of dead microorganisms, is aimed at denoting the amount of this microorganism necessary for the production of said amount of metabolites or of fractions of dead microorganisms.

The microorganism(s) may be included in a composition according to the invention in live, semi-active or inactivated, dead form.

It (they) may also be included in the form of fractions of cellular components or in the form of metabolites. The microorganism(s), metabolite(s) or fraction(s) may also be introduced in the form of a freeze-dried powder, of a culture supernatant and/or, where appropriate, in a concentrated form. I I

In the particular case of topical compositions, it may be advantageous to use these microorganisms in inactivated, or even dead, form.

A composition according to the invention may be in any of the galenical forms normally available for the method of administration selected.

The support may be of diverse nature depending on the type of composition under consideration.

With regard more particularly to the compositions intended for external topical administration, they may be aqueous, aqueous-alcoholic or oily solutions, solutions or dispersions of the lotion or serum type, emulsions with a liquid or semi-liquid consistency of the milk type, obtained by dispersion of a fatty phase in an aqueous phase (O/W) or vice versa (W/O), or suspensions or emulsions with a soft, semi-solid or solid consistency, of the cream type, an aqueous or anhydrous gel, microemulsions, microcapsules, microparticles, or vesicular dispersions of ionic and/or nonionic type.

These compositions are prepared according to the usual methods.

These compositions may in particular constitute cleansing, protection, treatment or care creams, skincare lotions, gels or foams, such as cleansing or disinfecting lotions, bath compositions or deodorant compositions.

The compositions according to the invention may also consist of solid preparations constituting cleansing soaps or bars.

They may also be used for the scalp in the form of solutions, creams, gels, emulsions, or foams, or else in the form of aerosol compositions also containing a pressurized propellant.

A topical composition according to the invention may advantageously be formulated in any galenical form suitable for haircare, in particular in the form of a lotion or shampoo, in particular an antidandruff lotion or shampoo, a conditioner, a haircare lotion, a hairstyling lacquer, cream or gel, a hairsetting lotion, a treating lotion, a dyeing (in particular oxidation dyeing) composition optionally in the form of a coloring shampoo, a hair-restructuring lotion, a permanent-wave composition, an anti-hairloss lotion or gel, an antiparasitic shampoo, a treating shampoo, in particular an antiseborrheic shampoo, or a disentangling lotion.

When the composition of the invention is an emulsion, the proportion of the fatty phase can range from 5% to 80% by weight, and preferably from 10% to 50% by weight, relative to the total weight of the composition. The oils, the emulsifiers and the coemulsifiers used in the composition in emulsion form are chosen from those conventionally used in the cosmetics and/or dermatological field. The emulsifier and the coemulsifier may be present, in the composition, in a proportion ranging from 0.3% to 30% by weight, and preferably from 0.5% to 20% by weight, relative to the total weight of the composition.

When the composition of the invention is an oily solution or gel, the fatty phase may represent more than 90% of the total weight of the composition.

In a known manner, the galenical forms devoted to topical administration may also contain adjuvants that are customary in the cosmetics, pharmaceutical and/or dermatological field, such as hydrophilic or lipophilic gelling agents, hydrophilic or lipophilic active agents, preservatives, antioxidants, solvents, fragrances, fillers, screens, odor-absorbers and dyestuffs. The amounts of these various adjuvants are those conventionally used in the field under consideration, and for example from 0.01% to 20% of the total weight of the composition. Depending on their nature, these adjuvants may be introduced into the fatty phase and/or into the aqueous phase.

As fats that can be used in the invention, mention may be made of mineral oils, for instance hydrogenated polyisobutene and liquid petroleum jelly, plant oils, for instance a liquid fraction of shea butter, sunflower oil and apricot kernel oil, animal oils, for instance perhydrosqualene, synthetic oils, in particular purcellin oil, isopropyl myristate and ethylhexyl palmitate, unsaturated fatty acids and fluoro oils, for instance perfluoropolyethers. Use may also be made of fatty alcohols, fatty acids, for instance stearic acid, and for instance waxes, in particular paraffin wax, carnauba wax and beeswax. Use may also be made of silicone compounds such as silicone oils and, for example, cyclomethicone and dimethicone, and silicone waxes, resins and gums.

As emulsifiers that can be used in the invention, mention may, for example, be made of glyceryl stearate, polysorbate 60, the mixture of cetylstearyl alcohol/oxyethylenated cetylstearyl alcohol comprising 33 mol of ethylene oxide, sold under the name Sinnowax AO^{®} by the company Henkel, the mixture of PEG-6/PEG-32/glycol stearate sold under the name Tefose^{®} 63 by the company Gattefosse, PPG-3 myristyl ether, silicone emulsifiers such as cetyl dimethicone copolyol and sorbitan monostearate or tristearate, PEG-40 stearate, and oxyethylenated (20 OE) sorbitan I I monostearate.

As solvents that can be used in the invention, mention may be made of low alcohols, in particular ethanol and isopropanol, and propylene glycol.

The composition of the invention may also advantageously contain a spring and/or mineral water, in particular chosen from Vittel water, waters from the Vichy basin and La Roche Posay water.

As hydrophilic gelling agents, mention may be made of carboxylic polymers such as carbomer, acrylic copolymers such as acrylate/alkyl acrylate copolymers, polyacrylamides, and in particular the mixture of polyacrylamide, C13-14-isoparaffin and laureth-7 sold under the name Sepigel 305^{®} by the company SEPPIC, polysaccharides, for instance cellulose derivatives, such as hydroxyalkylcelluloses, and in particular hydroxypropylcellulose and hydroxyethylcellulose, natural gums such as guar gum, carob gum and xanthan gum, and clays.

As lipophilic gelling agents, mention may be made of modified clays such as bentones, metal salts of fatty acids, such as aluminum stearates, and hydrophobic silica, or else ethylcellulose and polyethylene.

In the case of an oral use in accordance with the invention, the use of an ingestible support is preferred.

The ingestible support may be of diverse nature depending on the type of composition under consideration.

Tablets or lozenges, oral supplements in dry form and oral supplements in liquid form are thus in particular suitable as food or pharmaceutical supports.

They may, for example, be dietary supplements, the formulating of which can be carried out by the usual methods for producing, in particular, dragees, gel capsules, gels, emulsions, tablets, capsules and hydrogels for controlled release.

In particular, an extract of wolfberry according to the invention can be incorporated into any other forms of dietary supplements or of enriched foods, for example food bars or compacted or noncompacted powders. The powders may be diluted in water, a soda, milk products or soya derivatives, or be incorporated into food bars.

An extract of wolfberry of the invention may, moreover, be formulated with the usual excipients and components for such oral compositions or dietary supplements, i.e., in particular, fatty and/or aqueous components, humectants, thickeners, preservatives, texturing, flavoring and/or coating agents, antioxidants and dyes that are customary in the food sector.

The formulating agents and excipients for oral compositions, and in particular for dietary supplements, are known in this field and are not the subject of a detailed description herein.

The following are in particular suitable as food or pharmaceutical supports: milk, yogurt, cheese, fermented milks, milk-based fermented products, ices, cereal-based products or fermented-cereal-based products, milk-based powders, child and infant formulas, food products of the confectionery, chocolate or cereal type, animal feeds, in particular for domestic animals, tablets, gel capsules or lozenges, liquid bacterial suspensions, oral supplements in dry form and oral supplements in liquid form.

Irrespective of the method of administration under consideration, the effective amount of extract of wolfberry of the invention may also be advantageously combined with at least one other active agent.

Thus, a topical or oral composition or a combination according to the invention may also contain at least one active agent chosen from an antiseborrheic active agent, a moisturizing active agent, an antidandruff active agent and/or mixtures thereof.

Such a formulation advantageously makes it possible to amplify the beneficial effects of an extract of wolfberry of the invention.

The term "antiseborrheic active agent" is intended to mean a compound capable of regulating the activity of the sebaceous glands.

An antiseborrheic active agent suitable for the invention may in particular be chosen from retinoic acid, benzoyl peroxide, sulfur, vitamin B6 (or pyridoxine), selenium chloride, sea fennel; mixtures of extract of cinnamon, of tea and of octanoylglycine, such as Sepicontrol A5 TEA^{®} from SEPPIC ; the mixture of cinnamon, sarcosine and octanoylglycine sold in particular by the company SEPPIC under the trade name Sepicontrol A5^{®} ; zinc salts, such as zinc gluconate, zinc pyrrolidonecarboxylate (or zinc pidolate), zinc lactate, zinc aspartate, zinc carboxylate, zinc salicylate, zinc cysteate ; copper derivatives, and in particular copper pidolate such as Cuivridone^{®} from Solabia ; extracts of plants of the species *Arnica montana, Cinchona succirubra*, *Eugenia caryophyllata, Humulus lupulus, Hypericum perforatum*, *Mentha piperita, Rosmarinus officinalis, Salvia officinalis* and *Thymus vulgaris*, all sold, for example, by the company Maruzen; extracts of meadowsweet *(Spiraea ulmaria*), such as the product sold under the name Sebonormine^{®} by the company Silab ; extracts of the alga *Laminaria saccharina* such as the product sold under the name Phlorogine^{®} by the company Biotechmarine ; mixtures of extracts of salad burnet *(Sanguisorba officinalis*/*Poterium officinale)* roots, of ginger *(Zingiber officinalis)* rhizomes and of cinnamon (*Cinnamomum cassia)* bark, such as the product sold under the name Sebustop^{®} by the company Solabia ; linseed extracts, such as the product sold under the name Linumine^{®} by the company Lucas Meyer ; Phellodendron extracts such as those sold under the name Phellodendron extract BG^{®} by the company Maruzen or Oubaku liquid B by the company Ichimaru Pharcos ; mixtures of argan oil, of *Serenoa serrulata* (saw palmetto) extract and of sesame seed extract, such as the product sold under the name Regu SEB^{®} by the company Pentapharm; mixtures of extracts of willowherb, of *Terminalia chebula,* of nasturtium and of bioavailable zinc (microalgae), such as the product sold under the name Seborilys^{®} by the company Green Tech; extracts of *Pygeum afrianum,* such as the product sold under the name Pygeum afrianum sterolic lipid extract^{®} by the company Euromed ; extracts of *Serenoa serrulata,* such as those sold under the name Viapure Sabal^{®} by the company Actives International, or those sold by the company Euromed ; mixtures of extracts of plantain, of *Berberis aquifolium* and of sodium salicylate, such as the product sold under the name Seboclear^{®} by the company Rahn; clove extract, such as the product sold under the name Clove extract Powder^{®} by the company Maruzen ; argan oil, such as the product sold under the name Lipofructyl^{®} by Laboratoires Serobiologiques ; lactic protein filtrates, such as the product sold under the name Normaseb^{®} by the company Sederma ; extracts of the alga *Laminaria,* such as the product sold under the name Laminarghane^{®} by the company Biotechmarine ; oligosaccharides of the alga *Laminaria digitata,* such as the product sold under the name Phycosaccharide AC^{®} by the company Codif; sugar cane extracts, such as the product sold under the name Policosanol^{®} by the company Sabinsa ; sulfonated shale oil, such as the product sold under the name Ichthyol Pale^{®} by the company Ichthyol ; extracts of European meadowsweet (*Spiraea ulmaria*), such as the product sold under the name Cytobiol^{®} Ulmaire by the company Libiol; sebacic acid, in particular sold in the form of a sodium polyacrylate gel under the name Sebosoft^{®} by the company Sederma ; glucomannans extracted from konjac tuber and modified with alkylsulfonate chains, such as the product sold under the name Biopol Beta^{®} by the company Arch Chemical; extracts *of Sophora angustifolia,* such as those sold under the name Sophora powder^{®} or Sophora extract^{®} by the company Bioland ; extracts of *Cinchona succirubra* bark, such as the product sold under the name Red bark HS^{®} by the company Alban Muller; extracts of *Quillaja saponaria,* such as the product sold under the name Panama wood HS^{®} by the company Alban Muller ; glycine grafted onto an undecylenic chain, such as the product sold under the name Lipacide UG OR^{®} by the company SEPPIC ; the mixture of oleanolic acid and of nordihydroguaiaretic acid, such as the product sold in the form of a gel under the name AC.Net^{®} by the company Sederma ; phthalimidoperoxyhexanoic acid ; tri (C₁₂-C₁₃)alkyl citrate sold under the name Cosmacol^{®} ECI by the company Sasol; tri(C₁₄-C₁₅)alkyl citrate sold under the name Cosmacol^{®} ECL by the company Sasol; 10-hydroxydecanoic acid, and in particular mixtures of 10-hydroxydecanoic acid, of sebacic acid and of 1,10-decanediol, such as the product sold under the name Acnacidol^{®} BG by the company Vincience ; and mixtures thereof.

The antiseborrheic active agent is, for example, present in a content ranging from 0.1% to 10% by weight, preferably from 0.1% to 5% by weight, and preferentially from 0.5% to 3% by weight, relative to the total weight of the composition.

The term "antidandruff active agent" is intended to mean a compound capable of preventing the appearance of dandruff flakes, reducing the number thereof and/or making them completely disappear.

An antidandruff active agent suitable for the invention may in particular be chosen from:
- pyridinethione salts, in particular calcium, magnesium, barium, strontium, zinc, cadmium, tin and zirconium salts. The zinc salt of pyridinethione is particularly preferred. The zinc salt of pyridinethione is in particular sold under the name zinc omadine by the company Olin;
- trihalocarbamides of formula: 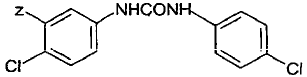 in which Z represents a halogen atom, such as chlorine, or a trihalo(C₁-C₄)alkyl group such as CF₃;
- triclosan represented by the formula: 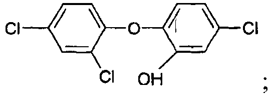
- azole compounds such as climbazole, ketoconazole, clotrinazole, econazole, isoconazole and miconazole;
- antifungal polymers such as amphotericin B or nystatin;
- selenium sulfides, in particular those of formula Sₓ Se ₈₋ₓ, x ranging from 1 to 7;
- sulfur in its various forms, cadmium sulfide, allantoin, coal tar or wood tar and derivatives thereof, in particular cade oil, salicylic acid, undecylenic acid, fumaric acid and allylamines such as terbinafine.

By way of preferred examples of antidandruff agents, mention may in particular be made of zinc pyrithione, salicylic acid and selenium disulfide, and mixtures thereof

A composition according to the invention advantageously comprises from 0.001% to 10% by weight of antidandruff agent(s), preferably from 0.1% to 5% by weight, even more preferably from 0.2% to 2% by weight, relative to the total weight of the composition.

A moisturizing active agent is an active agent capable of reducing the state of dryness of an epidermis.

The term "moisturizing active agent" is intended to mean:
- either a compound which acts on the barrier function, with a view to maintaining the moisturization of the stratum corneum, or an occlusive compound. Mention may be made of ceramides, sphingoid-based compounds, lecithins, glycosphingolipids, phospholipids, cholesterol and its derivatives, phytosterols (stigmasterol, β-sitosterol, campesterol), essential fatty acids, 1,2-diacylglycerol, 4-chromanone, pentacyclic triterpenes, petroleum jelly and lanolin;
- or a compound which directly increases the water content of the stratum corneum, such as urea and its derivatives, threalose and its derivatives, hyaluronic acid and its derivatives, glycerol, pentanediol, pidolates, serine, xylitol, lactic acid and sodium lactate, glyceryl polyacrylate, ectoin and its derivatives, chitosan, oligosaccharides and polysaccharides, cyclic carbonates, N-lauroylpyrrolidonecarboxylic acid and N-α-benzoyl-L-arginine;
- or a compound which activates the sebaceous glands, such as steroidal derivatives (including DHEA), and vitamin D and its derivatives.

These compounds may represent from 0.001% to 30%, and preferably from 0.01% to 20%, of the total weight of the composition according to the invention.

By way of illustration of the urea derivatives, mention may more particularly be made of hydroxyalkylurea derivatives, and in particular those described in document FR 2 877 222.

The topical or oral compositions, or combinations, according to the invention may also contain, as additional active agent, an active agent in particular intended to reinforce the cutaneous barrier.

By way of active agents that can be used, mention may be made of vitamins A, B3, B5, B6, B8, C, D, E, or PP, curcuminoids, carotenoids, polyphenol and inorganic compounds, sugars, amino acids, sulfur-containing amino acids, 3 and 6 polyunsaturated fatty acids, taurine and phytosterols.

In particular, use may be made of an antioxidant complex comprising flavonoids such as catechins, ubiquinones, extracts of coffee containing polyphenols and/or diterpenes, extracts of chicory, extracts of ginkgo biloba, proanthocyanidin-rich grape extracts, extracts of capsicum, soybean extracts, other sources of flavonoids having antioxidant properties, fatty acids, prebiotics, taurine, resveratrol, selenium amino acids, and glutathione precursors.

Among the flavonoids, catechins and OPCs (oligomeric proanthocyanidins) are preferably chosen.

At least one prebiotic or a mixture of prebiotics may also be involved. More particularly, these prebiotics may be chosen from oligosaccharides, produced from glucose, galactose, xylose, maltose, sucrose, lactose, starch, xylan, hemicellulose, inulin, gums of acacia type, for example, or a mixture thereof. More particularly, the oligosaccharide comprises at least one fructooligosaccharide. More particularly, this prebiotic may comprise a mixture of fructooligosaccharide and inulin.

In the topical galenic forms, use may more particularly be made, as hydrophilic active agents, of proteins or protein hydrolysates, amino acids, polyols, in particular C₂ to C₁₀ polyols such as glycerol, sorbitol, butylene glycol and polyethylene glycol, urea, allantoin, sugars and sugar derivatives, water-soluble vitamins, starch, bacterial extracts or plant extracts such as aloe vera.

As regards the lipophilic active agents, use may be made of retinol (vitamin A) and its derivatives, tocopherol (vitamin E) and its derivatives, ceramides, essential oils and unsaponifiable materials (tocotrienol, sesamine, gamma-oryzanol, phytosterols, squalenes, waxes, terpenes).

As active agents that can also be combined with the extract of wolfberry of the invention in an oral galenical formulation, any ingredient commonly used and/or permitted may also be considered.

By way of illustration, mention may be made of vitamins, minerals, essential lipids, trace elements, polyphenols, flavonoids, phytoestrogens, antioxidants such as lipoic acid and coenzyme Q10, carotenoids, prebiotics, proteins and amino acids, monosaccharides and polysaccharides, amino sugars, phytosterols and triterpenic alcohols of plant origin.

This may involve, in particular, vitamins A, C, D, E and PP and group B vitamins. Among the carotenoids, beta-carotene, lycopene, lutein, zeazanthin and astaxanthin are preferably chosen. The minerals and trace elements particularly used are zinc, calcium, magnesium, copper, iron, iodine, manganese, selenium and chromium(III). Among the polyphenol compounds, polyphenols from grape, from tea, from olive, from cocoa, from coffee, from apple, from blueberry, from elderberry, from strawberry, from cranberry and from onion are also in particular selected. Preferably, among the phytoestrogens, isoflavones in free or glycosylated form are selected, such as genistein, daidzein, glycitein or alternatively lignans, in particular those from flax and from *Schizandra chinensis.* The amino acids or the peptides and the proteins containing them, such as taurine, threonine, cysteine, tryptophan, methionine or carnitine. The lipids preferably belong to the group of oils containing monounsaturated and polyunsaturated fatty acids such as oleic acid, linoleic acid, alpha-linolenic acid, gamma-linolenic acid, stearidonic acid, long-chain fish omega-3 fatty acids such as EPA and DHA, conjugated fatty acids derived from plants or animals, such as CLAs (Conjugated Linoleic Acids) or petroselenic acid derived from coriander oil.

Thus, in particular when an extract of wolfberry of the invention is intended for oral administration, it may also be combined with at least one nutritional active agent chosen from lycopene, vitamin C, vitamin E and polyphenol compounds.

An extract of wolfberry of the invention or a combination according to the invention may also be combined with other nutritional active agents chosen from:
- anti-aging nutritional active agents, such as food antioxidants, nutrients with free-radical scavenging properties and cofactors of antioxidant endogenous enzymes, vitamins A, C and E, carotenoids, xanthophylls, isoflavones, certain minerals such as zinc, copper, magnesium, selenium, lipoic acid, coenzyme Q10, superoxide dismutase (SOD) or taurine. Among the anti-aging active agents, mention may in particular be made of the unsaponifiable fractions extracted from lipids of plant origin, aloe vera, native or hydrolyzed marine collagen, plant or marine oils rich in omega-3 and omega-6 fatty acids (including gamma-linolenic acid),
- photoprotective nutritional active agents such as: antioxidants and free-radical scavengers: vitamins A, C and E, carotenoids, xanthophylls, certain minerals such as zinc, copper, magnesium, selenium, coenzyme Q10, superoxide dismutase (SOD),
- nutritional ingredients with moisturizing or else immunomodulatory properties, such as extract of *Polypodium leucotomos,* and plant or marine oils rich in omega-3 and omega-6 fatty acids, including gamma-linolenic acid.

A cosmetic use of the invention may be implemented in particular by administering the cosmetic and/or dermatological compositions or combinations as defined above, according to the customary technique for using these compositions. For example: applications of creams, gels, sera, lotions, milks for removing makeup or of aftersun compositions to the keratin material such as the skin or dry hair, application of a hair lotion to wet hair or of shampoos, as regards topical application.

A cosmetic use according to the invention may thus be implemented by topical, for example daily, administration of a composition under consideration according to the invention.

A cosmetic use according to the invention may comprise a single administration. According to another embodiment, the administration is repeated, for example, 2 to 3 times daily for one day or more, and generally for a sustained period of at least 4 weeks, or even 4 to 15 weeks, with, where appropriate, one or more periods of interruption.

In the description and in the examples that follow, unless otherwise indicated, the percentages are percentages by weight and the ranges of values written in the form "between... and..." include the upper and lower limits specified.

The ingredients are mixed, before being formulated, in the order and under conditions that can be readily determined by those skilled in the art.

The content and the nature of the ingredients used in the compositions of the invention are adjusted by those skilled in the art in such a way as to not substantially affect the properties required for the compositions of the invention.

### FIGURES

Figure 1: illustrates the inhibition, with lacto-wolfberry (LWB), of the production of IL-6 induced by LPS in the murine macrophage cell line (RAW cells).
Figure 2: illustrates the inhibition, with lacto-wolfberry (LWB), of the activation of NF-κB induced by LPS in the human intestinal epithelial cell line (HT-29 stably transfected with an NF-κB reporter gene).
Figure 3: illustrates the loss of body weight after administration of TNBS (trinitrobenzenesulfonic acid), (mean +/- SEM).
Figure 4: illustrates the evaluation of the macroscopic appearance. Right-hand graph: mean +/- SEM; left-hand graph: individual values and median.
Figure 5: illustrates the evaluation of the histological appearance. Right-hand graph: mean +/- SEM; left-hand graph: individual values and median.
Figure 6: illustrates the ratio of COX-2 relative to the reference protein (actin). Right-hand graph: mean +/- SEM; left-hand graph: individual values and median.
Figure 7: illustrates the expression of pSTAT3. Right-hand graph: mean +/- SEM ; left-hand graph : individual values and median.
Figure 8: illustrates the expression of pro-inflammatory genes, such as TNFα, IL-6 and IL-1β, and of proteins such as KC, in a murine model of acute intestinal inflammation with or without lacto-wolfberry (LWB) supplementation.
Figure 9: illustrates the expression of antioxidant genes such as GPX-1, CAT1 and SOD2 in a murine model of acute intestinal inflammation with or without lacto-wolfberry (LWB) supplementation.
Figure 10: illustrates the antioxidant capacity. Right-hand graph: mean +/-SEM ; left-hand graph : individual values and median.
Figure 11: illustrates the expression of the gene encoding TNF-α in the liver of aged mice having received a lacto-wolfberry (LWB)-based dietary supplement, compared with the levels observed in the liver of normal aged mice (H20) and of adult mice (CTRL).

The examples hereinafter are given by way of nonlimiting illustration of the field of the invention.

### EXAMPLES

### Example 1

### Preparation of a milk extract of wolfberry or lacto-wolfberry

A milk extract of wolfberry is prepared from wolfberry fruit from the Ningxia province in China.

The whole fruits preferably used for the invention contain at least 110 mg, and preferably 150 mg, of zeaxanthin relative to 100 g of fruit. This extract can be prepared according to one of the methods described in example 1 or 4 of document WO 2005/092121.

The lacto-wolfberry thus obtained contains approximately 53% of wolfberry fruit, 30% of skimmed milk and 17% of maltodextrin or approximately 64% of wolfberry fruit and 36% of skimmed milk.

The extract thus obtained is used in the formulation specified hereinafter.

### Example 2

### Capsule

| | mg/capsule |
|---|---|
| Lacto-wolfberry (corresponding to 10.66 µg of zeaxanthin) | 13.7 |
| *Lactobacillus paracasei* ST 11 (CNCM I-2116) | 10¹⁰ cfu |
| Glycerol | 150 |
| Magnesium stearate | 0.02 |
| Natural flavor | qs |

One to three capsules can be taken per day.

### Example 3

### Reagents

Powdered lacto-wolfberry (LWB) originating from test batch No. WB03A1506H produced at the NRC (Nestlé Research Center; Lausanne) as described above and a powder of extract of wolfberry (batch No. WB03A1506H) were provided by J. Wang (FS, Lipids & Bioactives).

Lipopolysaccharide (LPS) from *E. coli* serotype 055:B5 was purchased from Sigma (St. Louis, Missouri). Human maternal milk (MM) was collected from various donors in good health, 20 days postpartum, after permission from an ethics committee. The keyhole limpet hemocyanin (KLH) comes from Sigma.

### Cell culture

The mouse monocyte/macrophage cell line RAW 264.7 (TIB-71 from ATCC, Manassas, Virginia, USA) was maintained in Dulbecco's modified Eagle medium (DMEM, Amimed, Bioconcept, Allschwill, Switzerland) supplemented with 10% heat-inactivated FCS (Amimed) and 1% of pencillin/streptomycin (Invitrogen, Paisley, UK) at 37°C in a 5% CO₂/air incubator.

The cells were passaged using trypsin/EDTA (Sigma, St-Louis, Missouri, USA). The cells of clone 34 of the HT-29 human colonic adenocarcinoma cell line (i.e. HT-29 cells stably transfected with an NF-κB reporter gene) were maintained in a DMEM (Bioconcept, Allschwill, Switzerland) with a high glucose content (4.5 g/l) containing 1% of stable L-glutamine and supplemented with 10% heat-inactivated (one hour at 56°C) FCS, 1% of penicillin/streptomycin (Sigma, St Louis, Missouri), 500 µg/ml of G418 (Invitrogen) and 100 µg/ml of Normocin (Invitrogen) at 37°C in a 5% CO₂/air incubator. The culture medium was changed every two days until the cell monolayers reached ∼ 90% confluence. The cells were subcultured using trypsin/EDTA (Sigma).

### Quantification of the expression of IL-6 induced by LPS

The RAW 264.7 cells were seeded in a proportion of 10⁴ cells/well in flat-bottomed 96-well plates (Nunc, Roskilde, Denmark) in a normal culture medium at 37°C I in a 5% CO₂/air incubator.

After three days of culture (i.e. the cells reaching ∼ 80% confluence), the cells were optionally stimulated with 0.5 µg/ml of *E. coli* LPS (055:B5, Sigma) in the absence or in the presence of samples of LWB (final concentration 0.1%-1%) for 24 hours in a normal culture medium. The cell culture supernatants were then recovered and used to quantify the production of IL-6. The levels of interleukin-6 (IL-6) in the cell supernatant were determined by ELISA (IL-6 Eli-pair murin, Diaclone, Besançon, France) in accordance with the manufacturer's instructions.

### NF-κB activity

The cells of HT-29 clone 34 were seeded in a proportion of 10⁴ cells/well in flat-bottomed 96-well plates (Nunc) in maintaining culture medium. After 3-4 days of culture (i.e. the cells reaching ∼ 80% confluence), the cells were washed in a phosphate buffered saline solution (Sigma), and then optionally stimulated with LPS (10 ng/ml + 5% maternal milk) or recombinant TNF-α (10 ng/ml; R&D systems, Oxon, England) in the absence or presence of samples of LWB for 24 hours in a DMEM containing 1% of penicillin/streptomycin.

The cell culture supernatants were then recovered and stored at +4°C overnight until analysis of the NF-κB activity.

Following NF-κB activation, the cells of HT-29 clone 34 secreted alkaline phosphatase (SEAP) into the culture supernatant. The release of SEAP was measured using a fluorescence detection method (Phospha-Light™ system) according to the manufacturer's instructions (Applied Biosystems, Bedford, USA). Briefly, the cell culture supernatants were incubated with a reaction buffer of the Phospha-Light™ system for 20 minutes in a white, flat-bottomed 96-well polypropylene plate (Greiner) and the luminescence was measured using the Spectrafluor Plus spectrometer (Tecan 8634 Hombrechtikon, Switzerland). The results were expressed in relative light units (RLU).

### TNBS-induced colitis model

The TNBS-induced colitis model is a model of acute inflammation induced by a chemical product, trinitrobenzenesulfonic acid (TNBS), at a dose of 150 mg/kg.

Ten mice per group were fed with a diet containing 1% of LWB (50 mg) for seven days before the induction of colitis and up to the time they were sacrificed. From day 1 to day 4 after TNBS administration, 50 mg of LWB were administered in order to compensate for the small food rations ingested owing to the acute intestinal inflammation. For each animal, the weight loss, the macroscopic and histological result, COX-2, pSTAT3, the expression of pro-inflammatory and antioxidant genes, and also the antioxidant capacity were evaluated. The macroscopic and histological evaluation is carried out on the basis of the criteria of Wallace *(Wallace et al.,* 1989) and Ameho (Ameho *et al.,* 1997). COX-2, which is the inducible form of cyclooxygenase, a 72kDa protein, is responsible for the inducible biosynthesis of prostaglandins in the event of acute inflammation. Stat3 is a key signal molecule for many cytokines, in particular for pro-inflammatory cytokines, such as IL-6.

### Study plan for evaluating immunomodulation in aged mice

Specific pathogen free male C57BL/6J mice (four weeks old) were purchased from Charles River Laboratories (France). The mice were placed under conventional conditions (cycles of 12 hours of day and night, at a temperature of 22°C and 56% humidity) and received, *ad libitum,* water and a semi-synthetic Kliba 3434 -based diet. Up until the age of 5 months, the mice were kept at 5 in a cage, and then they were each placed in individual cages. All these conditions and the handling of the animals were validated by the Nestlé Ethics Committee and the National Ethics Committee with the approval of the Conseil Vétérinaire Fédéral Suisse [Swiss Federal Veterinary Council]. At 21 months, the mice were randomly separated into two groups of 10. The nonsupplemented control mice (H20, n=10 per age group) and the mice supplemented with LWB (LWB, n=10 per age group) received a semi-synthetic diet (AIN-93). The LWB was administered in the form of a solution at 0.5% (w/v), added to their water, which was freshly prepared and changed every two days. During the test period (44 days), all the mice were able to eat and drink at will. The same study plan was repeated twice and was also performed with 8-month-old mice (n=8 per group).

In order to study the T-lymphocyte-dependent humoral response *in vivo* (production of antigen-specific antibodies), the aged mice were immunized, at day 15 of the test, by means of a subcutaneous injection (100 µl) of an inert keyhole limpet hemocyanin (KLH) antigen (Sigma) at 100 µg in 1% of alum (Brenntag Biosector, Frederikssund, Denmark). The delayed-type hypersensitivity (DTH) response was used as *an in vivo* measurement of cellular immunity. Ear thickness (ear swelling) measurements taken before and then from 24 hours to 8 days after induction, made it possible to determine the ability to generate a delayed-type hypersensitivity (DTH) response. Briefly, 7 days after the immunization of the mice with the keyhole limpet hemocyanin (i.e. test day 22), the DTH responses were induced by injecting the keyhole limpet hemocyanin booster antigen (10 µl of 0.5 µg/ml) into the right ear of each mouse. A carrier alone (saline solution = PBS) was injected into their left ear, and these ears served as an internal control for each animal. 24 hours after induction, and for the following 7 days, the two ears, nonstimulated (left) and stimulated (right), were measured.

The DTH responses (KLH-PBS) were expressed by the size of the swelling of the ear, i.e. the increase in ear thickness, according to the following formula: Δ ear thickness - [thickness of stimulated ear (right, KLH) - thickness of nonstimulated ear (left, PBS)], where Δ ear thickness = [ear thickness after stimulation - ear thickness before stimulation].

Blood samples were taken from the caudal vein at day 0, 15 and 29 and by cardiac puncture at day 44. The mice were sacrificed on day 44 of the test. During the autopsy, the liver was removed and a part was immediately frozen in liquid nitrogen. Samples were stored at -80°C for a more detailed analysis.

### Quantification of the titers of IgG2a antibodies corresponding to KLH

The volumes of IgG2a antibodies corresponding to KLH in the blood serum were determined via ELISA. Microtitration plates were coated with KLH (50 µl/well at 100 ng/ml) and incubated at 37°C for 3 hours. The free binding sites were blocked with an ELISA buffer for 1 hour at 37°C. Samples were then added and incubated at +4°C overnight. The bound antibodies were reacted for 1 hour at 37°C, with agitation, with a biotinylated anti-mouse IgG2a goat antibody (specific for the γ2a chain) from Southern Biotechnologies (Birmingham, USA). The plates were read at 450 nm after addition of the peroxidase substrate TMB from KPL. The anti-KLH IgG2a antibody titers were expressed in mean OD₄₅₀ₙₘ values.

### Gene expression

Liver samples were transferred into 1 ml of RNA lysis buffer (Macherey-Nagel, Düren, Germany) and homogenized using a Ribolyzer (Hybaid, Waltham, MA, USA) programmed according to the following parameters: power 6 for 20 seconds.

The RNA extraction was carried out using a commercially available kit (NucleoSpin RNA II kit; Macherey-Nagel, Düren, Germany). The amount of RNA was evaluated using the Ribogreen RNA Quantitation kit (Molecular Probes; Eugene, Oregon USA), and the RNA quality was evaluated with the Agilent RNA 6000 Nano LabChip kit (Agilent Technologies, Palo Alto, USA). The total RNA (2 µg) was subjected to reverse transcription using the Multiscribe reverse transcriptase system, according to the manufacturer's instructions (Applied Biosystems, Biosystems; Rokreutz, Switzerland).

Custom-made low-density arrays with 48 TaqMan probes (loading capacity: 8 samples per array in technical monoplicates) were purchased from Applied Biosystems (Foster City, USA) and used according to the manufacturer's instructions. Gene expression was calculated using the ΔΔCt relative quantification method with SDS 2.2.2 software (Applied Biosystems). The resulting cycle threshold (Ct) values were exported into MS Excel (Microsoft, USA) for further analysis. The ΔCt value (i.e. Ct value of the target gene - Ct value of the GAPDH housekeeping gene) was first calculated and then the relative mRNA expression was determined using the following formula: 2^{-ΔCt} × 10⁶.

### General statistical analysis

Data were analyzed by mean +/- SEM or SD (standard deviation), and the Student's T Test (unpaired) or two-way Anova model where appropriate. Probability values of less than 5% were considered to be significant.

### Results

### In vitro experiment demonstrating the anti-inflammatory properties of lacto-wolfberry

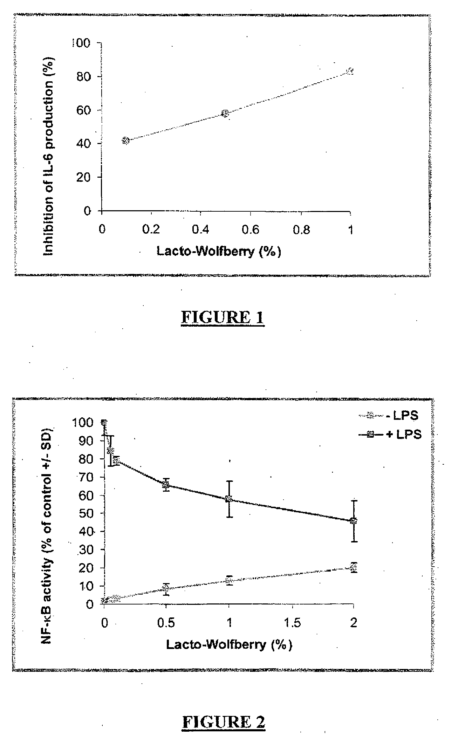

A solution of lacto-wolfberry (LWB) *inhibited, in vitro,* the LPS-induced release of the pro-inflammatory cytokine IL-6 in a murine macrophage cell line (figure 1) and the LPS-induced activation of NF-κB in human intestinal epithelial cells (figure 2).

### In vivo experiments demonstrating the anti-inflammatory and immunomodulatory effects of lacto-wolfberry

### Pathological acute inflammation

An LWB-based dietary supplement demonstrated strong anti-inflammatory properties in a murine model of acute intestinal inflammation.

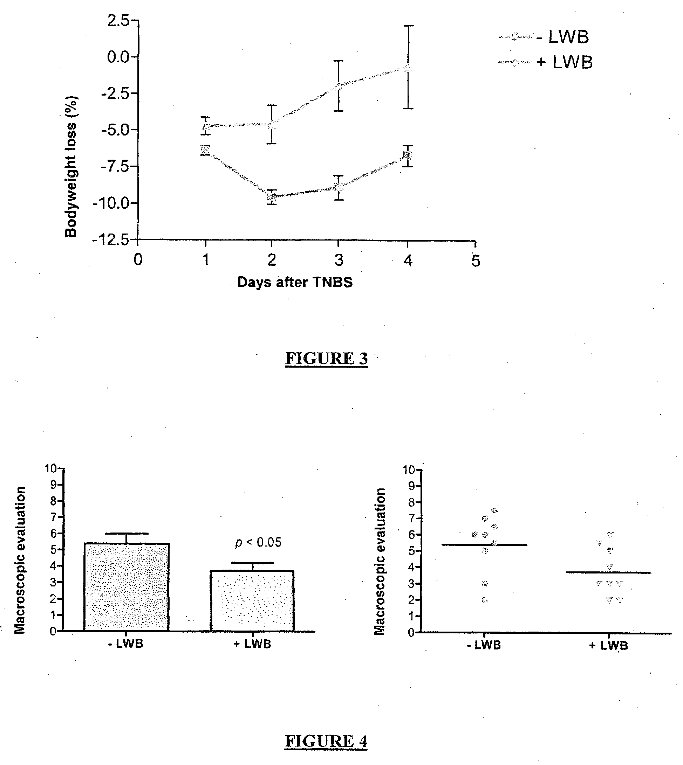

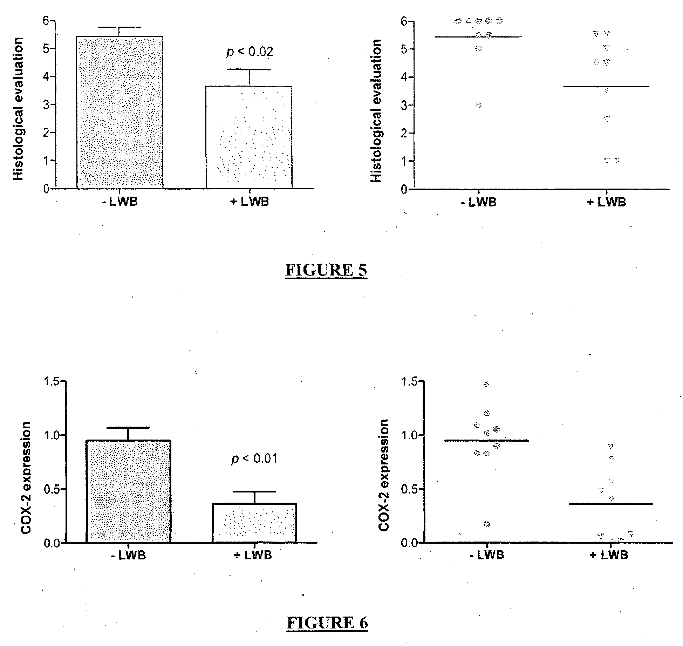

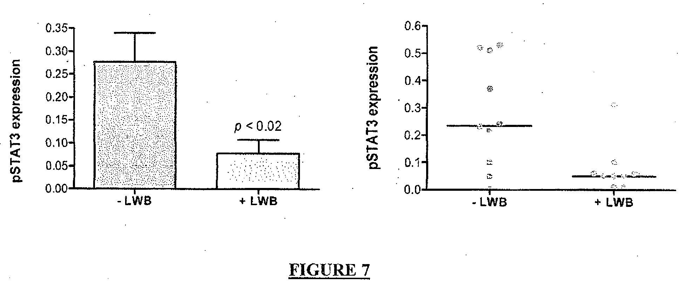

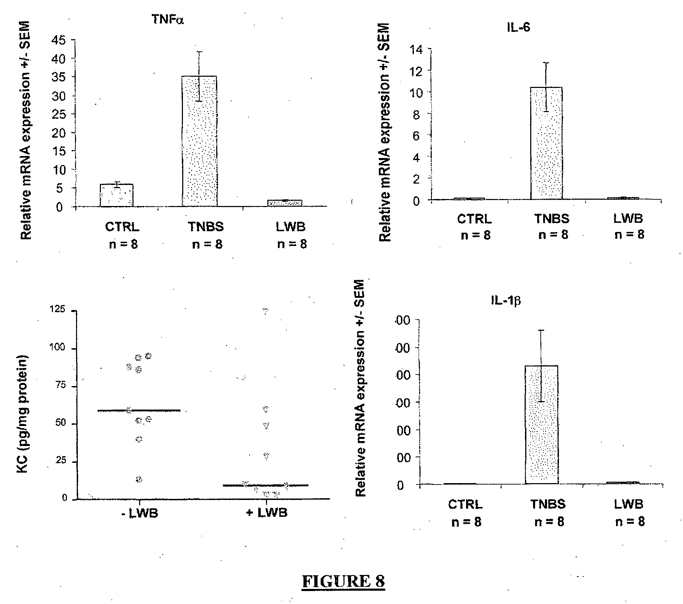

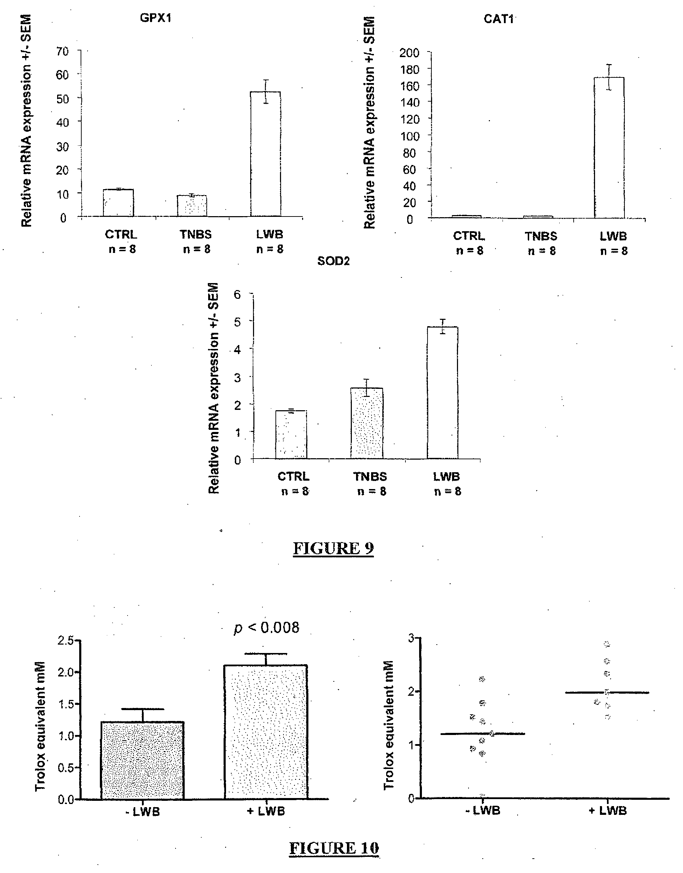

Oral administration of 1% LWB resulted in an improvement of bodyweight loss (figure 3) and in macroscopic and histological lesions (figures 4 and 5) and a significant reduction of COX-2 and pSTAT3 expression levels (figures 6 and 7). In addition, expression of pro-inflammatory genes, such as TNFα, IL-6 and IL-1β, and of proteins such as KC in the colon were reduced, compared to those of the control group (figure 8). In parallel, LWB induced a strong expression of antioxidant genes such as GPX-1, CAT1 and SOD2 (figure 9) in the colon and increased antioxidant defenses in the plasma (figure 10).

### Physiological age-related low-grade inflammation

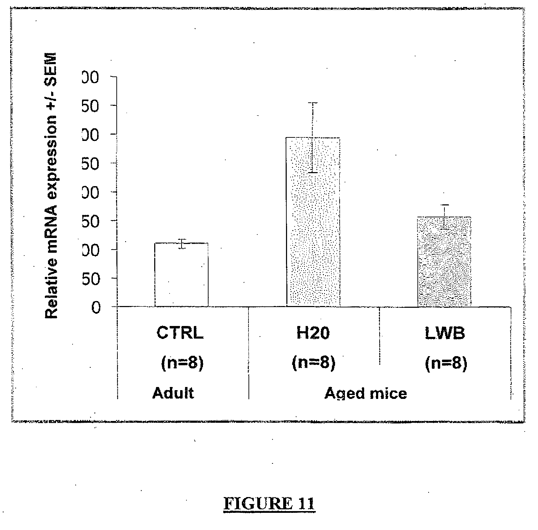

An LWB-based dietary supplement (0.5% in the drinking water) induced a reduction in inflammation-related genes in the liver of aged mice, to levels observed in adult mice (figure 11: for example, the gene encoding TNF-α).

## Claims

1. A cosmetic use of an effective amount of an extract of wolfberry as an agent for preventing and/or treating aesthetic scalp disorders, wherein said extract of wolfberry is obtained by extraction of wolfberry fruit of at least one plant of the species *Lycium barbarum* in a liquid medium containing milk or milk proteins, and wherein the aesthetic scalp disorder is an inflammation-related dandruff condition of the scalp.

2. The use as claimed in the preceding claim, wherein said extract of wolfberry contains at least zeaxanthin or a derivative thereof and/or *Lycium barbarum* polysaccharide.

3. The use as claimed in any one of the preceding claims, wherein said extract of wolfberry contains from 0.01% to 0.30% by weight, in particular from 0.03% to 0.12% by weight, or even from 0.06% to 0.10% by weight, of zeaxanthin or derivatives, including zeaxanthin dipalmitate and other zeaxanthin derivatives, relative to the total weight thereof.

4. The use as claimed in any one of the preceding claims, wherein said extract of wolfberry is used topically or orally.

5. The use as claimed in any one of the preceding claims, **characterized in that** the extract of wolfberry is combined with at least an effective amount of at least one probiotic microorganism, of a fraction thereof or of a metabolite thereof.

6. The use as claimed in the preceding claim, **characterized in that** the microorganism is chosen from *Lactobacillus johnsonii, Lactobacillus reuteri, Lactobacillus paracasei, Lactobacillus casei, Bifidobacterium bifidum, Bifidobacterium breve, Bifidobacterium longum, Bifidobacterium animalis, Bifidobacterium lactis, Bifidobacterium infantis, Bifidobacterium adolescentis* or *Bifidobacterium pseudocatenulatum,* a fraction thereof or a metabolite thereof, and mixtures thereof.

7. The use as claimed in claim 5 or 6, **characterized in that** the microorganism is of the species *Lactobacillus paracasei,* a fraction thereof or a metabolite thereof, and preferably the *Lactobacillus paracasei* strain deposited on January 12, 1999, under the designation CNCM I-2116, a fraction thereof or a metabolite thereof.

8. The use as claimed in any one of claims 5 to 7, wherein said microorganism is used in a proportion of from 0.0001% to 20% by weight, in particular from 0.001% to 15%, and more particularly from 0.01% to 10% by weight, and especially from 0.1% to 2% by weight, relative to the total weight of the composition containing it.

9. An effective amount of at least one extract of wolfberry for use in preventing and/or treating inflammation of the scalp, wherein said extract of wolfberry is obtained by extraction of wolfberry fruit of at least one plant of the species *Lycium barbarum* in a liquid medium containing milk or milk proteins.

10. A cosmetic and/or dermatological composition, for use in preventing and/or treating an inflammation of the scalp, comprising, in a physiologically acceptable medium, at least an effective amount of at least one extract of wolfberry, optionally in combination with an effective amount of at least one active agent chosen from a moisturizing active agent, an antiseborrheic active agent, an antidandruff active agent, and mixtures thereof; wherein said extract of wolfberry is obtained by extraction of wolfberry fruit of at least one plant of the species *Lycium barbarum* in a liquid medium containing milk or milk proteins.

11. The composition for use as claimed in the preceding claim, for preventing and/or treating an inflammation-related dandruff condition of the scalp.

## Patentansprüche

1. Kosmetische Verwendung einer wirksamen Menge eines Extrakts von Bocksdorn als ein Mittel zur Vorbeugung und/oder Behandlung von ästhetischen Störungen der Kopfhaut, wobei der Extrakt von Bocksdorn durch Extraktion von Bocksdorn-Frucht von mindestens einer Pflanze der Spezies *Lycium barbarum* in einem flüssigen Medium, das Milch oder Milchproteine enthält, erhalten wird, und wobei die ästhetische Störung der Kopfhaut ein mit Entzündung in Zusammenhang stehender Zustand von Schuppen der Kopfhaut ist.

2. Verwendung wie in dem vorstehenden Anspruch beansprucht, wobei der Extrakt von Bocksdorn mindestens Zeaxanthin oder ein Derivat davon und/oder *Lycium barbarum*-Polysacchalid enthält.

3. Verwendung wie in einem der vorstehenden Ansprüche beansprucht, wobei der Extrakt von Bocksdorn 0,01 Gew.-% bis 0,30 Gew.-%, insbesondere 0,03 Gew.-% bis 0,12 Gew.-% oder sogar 0,06 Gew.-% bis 0,10 Gew.-% Zeaxanthin oder Derivate, einschließend Zeaxanthindipalmitat und weitere Zeaxanthin-Derivate, relativ zum Gesamtgewicht davon, enthält.

4. Verwendung wie in einem der vorstehenden Ansprüche beansprucht, wobei der Extrakt von Bocksdorn topisch oder oral verwendet wird.

5. Verwendung wie in einem der vorstehenden Ansprüche beansprucht, **dadurch gekennzeichnet, dass** der Extrakt von Bocksdorn mit mindestens einer wirksamen Menge von mindestens einem probiotischen Mikroorganismus, einer Fraktion davon oder einem Metaboliten davon kombiniert wird.

6. Verwendung wie in dem vorstehenden Anspruch beansprucht, **dadurch gekennzeichnet, dass** der Mikroorganismus aus *Lactobacillus johnsonii, Lactobacillus reuteri*, *Lactobacillus paracasei, Lactobacillus casei, Bifidobacterium bifidum*, *Bifidobacterium breve, Bifidobacterium longum*, *Bifidobacterium animalis, Bifidobacterium lactis, Bifidobacterium infantis, Bifidobacterium adolescentis* oder *Bifidobacterium pseudocatenulatum*, einer Fraktion davon oder einem Metaboliten davon, und Gemischen davon ausgewählt ist.

7. Verwendung wie in Anspruch 5 oder 6 beansprucht, **dadurch gekennzeichnet, dass** der Mikroorganismus die Spezies *Lactobacillus paracasei,* eine Fraktion davon oder ein Metabolit davon, und bevorzugt der *Lactobacillus paracasei*-Stamm, der unter der Bezeichnung CNCM I-2116 am 12. Januar 1999 hinterlegt wurde, eine Fraktion davon oder ein Metabolit davon ist.

8. Verwendung wie in einem der Ansprüche 5 bis 7 beansprucht, wobei der Mikroorganismus in einem Anteil von 0,0001 Gew.-% bis 20 Gew.-%, insbesondere 0,001 Gew.-% bis 15 Gew.-% und stärker besonders 0,01 Gew.-% bis 10 Gew.-% und speziell 0,1 Gew.-% bis 2 Gew.-%, relativ zum Gesamtgewicht der Zusammensetzung, welche ihn enthält, verwendet wird.

9. Wirksame Menge von mindestens einem Extrakt von Bocksdorn zur Verwendung in der Vorbeugung und/oder Behandlung von Entzündung der Kopfhaut, wobei der Extrakt von Bocksdorn durch Extraktion von Bocksdorn-Frucht von mindestens einer Pflanze der Spezies *Lycium barbarum* in einem flüssigen Medium, das Milch oder Milchproteine enthält, erhalten wird.

10. Kosmetische und/oder dermatologische Zusammensetzung zur Verwendung in der Vorbeugung und/oder Behandlung einer Entzündung der Kopfhaut, umfassend in einem physiologisch verträglichen Medium mindestens eine wirksame Menge von mindestens einem Extrakt von Bocksdorn, gegebenenfalls in Kombination mit einer wirksamen Menge von mindestens einem Wirkstoff, welcher aus einem Feuchthalte-Wirkstoff, einem Wirkstoff gegen Seborrhoe, einem Wirkstoff gegen Schuppen und Gemischen davon ausgewählt ist; wobei der Extrakt von Bocksdorn durch Extraktion von Bocksdorn-Frucht von mindestens einer Pflanze der Spezies *Lycium barbarum* in einem flüssigen Medium, das Milch oder Milchproteine enthält, erhalten wird.

11. Zusammensetzung zur Verwendung wie in dem vorsehenden Anspruch beansprucht zur Vorbeugung und/oder Behandlung eines mit Entzündung in Zusammenhang stehenden Zustandes von Schuppen der Kopfhaut.

## Revendications

1. Utilisation cosmétique d'une quantité efficace d'un extrait de Wolfberry comme agent pour prévenir et/ou traiter les désordres esthétiques du cuir chevelu, dans laquelle ledit extrait de Wolfberry est obtenu par extraction de fruit de Wolfberry d'au moins un végétal de l'espèce *Lycium barbarum* dans un milieu liquide contenant du lait ou des protéines du lait, et dans laquelle le désordre esthétique du cuir chevelu est un état pelliculaire du cuir chevelu lié à une inflammation.

2. Utilisation selon la revendication précédente, dans laquelle ledit extrait de Wolfberry contient au moins de la zéaxanthine ou l'un de ses dérivés et/ou du polysaccharide de *Lycium barbarum.*

3. Utilisation selon l'une quelconque des revendications précédentes, dans laquelle ledit extrait de Wolfberry contient de 0,01 à 0,30 % en poids, en particulier de 0,03 à 0,12 % en poids, voire de 0,06 à 0,10 % en poids de zéaxanthine ou dérivés, incluant le dipalmitate de zéaxanthine et d'autres dérivés de la zéaxanthine, par rapport à son poids total.

4. Utilisation selon l'une quelconque des revendications précédentes, dans laquelle ledit extrait de Wolfberry est mis en oeuvre par voie topique ou orale.

5. Utilisation selon l'une quelconque des revendications précédentes, **caractérisée en ce que** l'extrait de Wolfberry est associé à au moins une quantité efficace d'au moins un microorganisme probiotique, de l'une de ses fractions ou de l'un de ses métabolites.

6. Utilisation selon la revendication précédente, **caractérisée en ce que** le microorganisme est choisi parmi les *Lactobacillus johnsonii, Lactobacillus reuteri, Lactobacillus paracasei, Lactobacillus casei, Bifidobacterium bifidum, Bifidobacterium breve, Bifidobacterium longum, Bifidobacterium animalis, Bifidobacterium lactis, Bifidobacterium infantis, Bifidobacterium adolescentis* ou *Bifidobacterium pseudocatenulatum,* l'une de leurs fractions ou l'un de leurs métabolites et leurs mélanges.

7. Utilisation selon la revendication 5 ou 6, **caractérisée en ce que** le microorganisme est de l'espèce *Lactobacillus paracasei,* l'une de ses fractions ou l'un de ses métabolites, et de préférence la souche *Lactobacillus paracasei* déposé le 12 janvier 1999 sous la désignation CNCM I-2116, l'une de ses fractions ou l'un de ses métabolites.

8. Utilisation selon l'une quelconque des revendications 5 à 7, dans laquelle ledit microorganisme est mis en oeuvre à raison de 0,0001 à 20 % en poids, en particulier de 0,001 à 15 % et plus particulièrement de 0,01 à 10 % en poids et notamment de 0,1 à 2 % en poids par rapport au poids total de la composition le contenant.

9. Quantité efficace d'au moins un extrait de Wolfberry, utile pour prévenir et/ou traiter une inflammation du cuir chevelu, dans laquelle ledit extrait de Wolfberry est obtenu par extraction de fruit de Wolfberry d'au moins un végétal de l'espèce *Lycium barbarum* dans un milieu liquide contenant du lait ou des protéines du lait.

10. Composition cosmétique et/ou dermatologique, utile pour prévenir et/ou traiter une inflammation du cuir chevelu, comprenant dans un milieu physiologiquement acceptable, au moins une quantité efficace d'au moins un extrait de Wolfberry, facultativement en association avec une quantité efficace d'au moins un principe actif choisi parmi un principe actif hydratant, un principe actif anti-séborrhéique, un principe actif antipelliculaire, et leurs mélanges ; dans laquelle ledit extrait de Wolfberry est obtenu par extraction de fruit de Wolfberry d'au moins un végétal de l'espèce *Lycium barbarum* dans un milieu liquide contenant du lait ou des protéines du lait.

11. Composition pour une utilisation selon la revendication précédente, pour prévenir et/ou traiter un état pelliculaire du cuir chevelu lié à une inflammation.
